# EUROPEAN PATENT APPLICATION

(11) **EP 1 913 950 A1**
(43) Date of publication of application: **23.04.2008**
(21) Application number: 07020280.9
(22) Date of filing: 17.10.2007
(51) Int. Cl.: A61K 36/258

(54) **Method for producing ginseng fruit and ginseng flower stalk with high content of ginsenoside**

(30) Priority: 18.10.2006 KR 20060101539; 06.07.2007 KR 20070068231; 27.09.2007 KR 20070097500
(71) Applicant: Seong, In-Hwan, Gangseo-gu Seoul 157-016 (KR)
(72) Inventor: Seong, In-Hwan, Gangseo-gu Seoul 157-016 (KR)
(74) Representative: Schweitzer, Klaus

(57) **Abstract**

Disclosed is a method for producing ginseng fruits and flower stalks with a high content of ginsenosides. More particularly, the present invention provides a method for producing ginseng fruits and flower stalks with a high content of ginsenoside Rb1, ginsenoside Rb2, ginsenoside Rc, ginsenoside Rd, ginsenoside Re and other ginsenoside ingredients. According to the present invention, the ginseng fruits and flower stalks have considerably increased contents of ginsenoside Re, Rd and Rb2 when compared with common red ginseng roots and/or black ginseng roots. In addition, the present invention provides ginseng fruits and flower stalks based products with a high content of, especially, ginsenoside Rb1, ginsenoside Rb2, ginsenoside Rc, ginsenoside Rd and/or ginsenoside Re.

## Description

### BACKGROUND OF THE INVENTION

Disclosed is a method for producing ginseng fruits and flower stalks with a high content of ginsenosides. More particularly, the present invention provides a method for producing ginseng fruits and flower stalks with a high content of ginsenoside Rb1, ginsenoside Rb2, ginsenoside Rc, ginsenoside Rd, ginsenoside Re and other ginsenoside ingredients.

According to the present invention, the ginseng fruits and flower stalks have considerably increased contents of ginsenoside Re, Rd and Rb2 when compared with common red ginseng roots and/or black ginseng roots. In addition, the present invention provides ginseng fruits and flower stalks based products with a high content of, especially, ginsenoside Rb1. ginsenoside Rb2, ginsenoside Rc, ginsenoside Rd and/or ginsenoside Re.

### FIELD OF THE INVENTION

The present invention relates to methods for cultivation of ginseng fruits and ginseng flower stalks with high content of ginsenoside, more particularly, to a method for production of ginseng fruits and ginseng flower stalks with high contents of ginsenoside ingredients such as ginsenoside Rb1, ginsenoside Rb2, ginsenoside Rc, ginsenoside Rd, ginsenoside Re and others,

### DESCRIPTION OF THE RELATED ART

Ginseng, which is named Panax ginseng C.A. Meyer according to the rule of Nomenclature as one of semi-shade type perennial plants and belongs to Araliaceae, is well known in a variety of medical literatures published in China and Korea to have functions and performances beneficial to human bodies.

As a result of current researches and investigations about ginseng, ginseng is found to contain plenty of ginsenoside also called 'Saponin' and is often applied in manufacturing health foods and/or health supplement foods recently besides traditional uses thereof such as medicines.

Ginseng cultivation usually takes at least four (4) years and, preferably, six (6) years to produce the best quality of ginseng. That is, ginseng needs a long period of time until it is completely grown and the harvest of the ginseng is usually less than the expected to be cultivated amount thereof due to different reasons including infestation with various insects and damage from diseases during cultivation, thus, leading to an increase in price and requirement of much time to cultivate ginseng fruits.

Ginseng has roots and rhizomes under the ground, and stems, leaves and fruits above the ground. More particularly, ginseng has roots including fine roots, lateral roots and main roots, rhizomes, stems which continue from the rhizomes, leaves extended from the stems, flowers and fruits produced at ends of the stems, and small branches to connect the fruits and stems called flower stalks.

A ginseng plant begins to blossom in the third (3) year of growth, and generates green fruits in May which, in turn, become ripe and turn red in the middle of July which are then harvested.

As ginseng growers mostly wish to obtain and utilize ginseng roots, in order to promote growth of the roots rather than reproductive growing of fruits, they generally pick and discard fruits when the ginseng plant begins to bear the fruits. In this case, they partially cut peduncles of the plant and discard them together with the fruits.

Ginsenoside refers to saponin contained in the ginseng plant. A variety of ginsenoside ingredients are contained in the ginseng plant. The ginsenoside ingredients of the ginseng plant include, for example, Panaxadiol PD, Panaxatriol PT, Oleanane species, etc. The ginseng plant also contains non-ginsenoside materials including carbohydrates such as starch, anti-oxidant aromatic compounds of polyacetylene, Gomisin N-A to protect human livers, acidic peptides with insulin-like functions etc.

Ginsenosides have pharmacological activities principally effective to the central nervous system, endocrine system, immune system, metabolic system, etc. Additionally, the ginsenosides are well known to have a plurality of advantages including: (1) a great lipolysis ability and promotion of nutrition absorption and digestion; (2) promotion of metabolism by activating enzymes in cells; (3) refreshment and improvement in fatigue, weakness, anorexia nervosa and the like; and (4) acceleration of serum protein synthesis, etc.

The present inventors found and suggested that ginsenoside Re shows central nervous inhibition, DNA and RNA behavior promotion, plasmin activation and promotion of adrenocorticotropic hormone release, ginsenoside Rd promotes release of adrenal cortex hormone, and ginsenoside Rb2 exhibits central nervous inhibition, DNA and RNA behavior promotion, plasmin activation, promotion of adrenocorticotropic hormone release and anti-diabetic effect.

Yet, dispite the content of ginsenosides in the fruit and flower stalk, such ginseng fruits and ginseng flower stalks are substantially discarded as waste after removal thereof from the ginseng plant.

### SUMMARY OF THE INVENTION

One object of the present invention is to provide a method for production of ginseng fruits and flower stalks with a high content of ginsenoside ingredients, for example, ginsenoside Rb1, ginsenoside Rb2, ginsenoside Rc, ginsenoside Rd, ginsenoside Re and others.

In one embodiment the present invention provides a method for producing ginseng fruits and flower stalks with a high content of ginsenoside by means of: treating the ginseng fruits and flower stalks by light irradiation; subjecting the light-treated fruits and flower stalks to water vapor then drying the vapor treated product optionally repeating steps b + c once or several times: and then treating the dried product in a solution containing at least one ginsenoside decomposer.

According to the present invention, the produced ginseng fruits and flower stalks contain many ginsenoside ingredients which may appropriately be added to health supplement foods and/or used to manufacture ginsenoside-rich health supplement foods.

The ginseng fruits and flower stalks with the high content of ginsenosides according to the present invention are preferably obtained from the ginseng fruits and flower stalks discarded in farm fields and processed into ginsenoside-rich ginseng products.

The ginseng fruits and flower stalks of the present invention may undergo a concentration process using a concentrator to produce a ginsenoside enriched extract e.g. with a high amount of ginsenoside Rb1, ginsenoside Rb2, ginsenoside Rc, ginsenoside Rd, ginsenoside Re, etc.

The present invention can use ginseng fruits to produce the ginseng fruits and flower stalks with high contents of ginsenoside Rb1. ginsenoside Rb2, ginsenoside Rc, ginsenoside Rd, ginsenoside Re and other ginsenoside ingredients besides traditionally used ginseng roots, and therefore, (can contribute to an increase in income of the ginseng farmers.

### DETAILED DESCRIPTION OF THE INVENTION

### 1. Steaming and drying the ginseng fruits and flower stalks after light irradiation.

In order to increase the content of the ginsenoside ingredients in the fruits and flower stalks, they are preferably subjected to light irradiation after gathering the fruits and flower stalks from ginseng farm fields.

Such light irradiation can be sun light containing all of ultraviolet (UV) rays, visible rays and infrared rays.

Preferably, such light irradiation is selected from at least one of UV rays, visible rays and infrared rays.

UV rays used in the present invention preferably comprise UV rays from 10 to 397 nm wavelength.

The visible rays used in the present invention preferably comprise visible rays from 380 to 770 nm wavelength.

The infrared rays used in the present invention preferably comprise infrared rays from 0.75 µm 1,000 µm wavelength.

The light irradiation of the ginseng fruits and flower stalks is preferably performed for about 1 to 24 hours. When the irradiation time is less than 1 hour, the content of ginsenoside in the ginseng fruits and flower stalks is only slightly increased. On the other hand, if the irradiation time exceeds 24 hours, there is no longer a remarkably improved effect on the increase in the ginsenoside content and, conversely, there is a likelyhood of reducing other nutritive substances contained in the fruits and flower stalks. Therefore, the light irradiation is preferably performed for 1 to 24 hours for producing the ginseng fruits and flower stalks with high content of ginsenoside of the present invention.

In order to increase the ginsenoside content and/or obtain a variety of ginsenoside ingredients during the production of the ginseng fruits and flower stalks with high content of ginsenoside of the present invention, the ginseng fruits and flower stalks are treated in a steam process.

The steam process is performed by using water vapor at 90 to 120 °C for 1 to 3 hours.

The steam process of the ginseng fruits and flower stalks is performed at least one a preferably more than once, e.g. two, three or four times.

More preferably, the steam process of the ginseng fruits and flower stalks is performed by using water vapor at 90 to 120 °C four (4) times for 1 to 3 hours per time.

The steamed fruits and flower stalks are dried to remove the water.

In case of applying the steam process several times, the drying process is usually carried out immediately after completing each individual steam process.

The drying process after the steam process for the fruits and flower stalks is carried out at 65 to 75°C, preferably at 65 and 70 °C until the remaining water on the outer surface of the fruits and flower stalks has been removed.

The drying process for the steamed product of the fruits and flower stalks may be carried out using general methods commonly known in the art. One example of these method may include any one selected form airdrying at ordinary temperature, heat drying with hot air, freeze drying at low temperature etc.

### 2. Treatment of the ginseng fruits in a solution containing a ginsenoside decomposer, after the steam and drying processes.

Ginseng contains various kinds of ginsenoside ingredients, which are not directly absorbable in a human body, Such ginsenosides must first be transformed into substances capable of being absorbed in the human body, e.g. by decomposers such as body enzymes.

Thus, according to the present invention the irradiated, steam treated and dried ginseng fruits and flower stalks are treated in a solution containing at least one ginsenoside decomposer.

The ginsenoside decomposer may include any one or more compounds selected form Hasegawa bacteria (bacteria having decomposition character, discovered by Hasegawa(JP)), α-glucosidase, α-Rhamnosidase, soyasapogenol B and the like,

The solution containing the ginsenoside decomposer is prepared by adding the ginsenoside decomposer(s) to pure water in an amount of 1 to 20 % by weight (abbrev. to "wt.%") of total weight of the pure water.

If the amount is less than 1 wt.% of the total weight of the pure water, the content of ginsenoside and/or ginsenoside ingredients which are easily absorbable in the human body is not increased. On the other hand, when the amount exceeds 20 wt.%, there is no further remarkably beneficial effect in terms of increase in the content of ginsenoside and/or ginsenoside ingredients which are easily absorbable in the human body, and there may be adverse effects to other ingredients contained in the ginseng fruits and flower stalks which is thus not preferable. Therefore, the ginsenoside decomposer is preferably added in an amount of from 1 to 20 wt.% to total weight of the pure water to prepare the solution, in which the ginseng fruits and flower stalks are treated after the light irradiation and the steam and drying processes.

The fruits and flower stalks treated by the light irradiation and the steam and drying processes may be subjected to the treatment process with the ginsenoside decomposer(s) for 10 minutes to 24 hours.

The present invention optionally comprises at least one additional treatment selected from ultrasonic treatment, low frequency treatment, electric field treatment and magnetic field treatment after any one of the above steps, in order to produce the ginseng fruits and flower stalks containing plenty of ginsenoside ingredients.

The ultrasonic treatment is performed at not less than 20 kHz for more than 10 minutes.

Preferably, the ultrasonic treatment is performed at 20 to 50 kHz for 10 minutes to 2 hours.

According to the present invention the ultrasonic treatment under the above condition was demonstrated to provide fruits and flower stalks with an even further increased content of ginsenoside ingredients,

The low frequency treatment is performed at not more than 10 kHz for more than 30 minutes.

Preferably, the low frequency treatment is performed at 5 to 10 kHz for 30 minutes to 2 hours.

According to the present invention the low frequency treatment under the above condition was demonstrated to provide fruits and flower stalks with an even further increased content of ginsenoside ingredients.

The electric field treatment is performed in an electric field of not less than 5 kV for more than 30 seconds.

Preferably, the electric field treatment is performed at 5 to 20 kV for 30 seconds to 5 minutes.

According to the present invention the electric field treatment, the electric field treatment under the above condition was demonstrated to provide fruits and flower stalks with an even further increased content of ginsenoside ingredients.

The magnetic field treatment is performed in a magnetic field of not less than 5 Gauss (G) for more than 30 minutes.

Preferably, the magnetic field treatment is performed at 5 to 30 G for 30 minutes to 1 hour.

According to the present invention the magnetic field treatment, the magnetic field treatment under the above condition was demonstrated to provide fruits and flower stalks with an even further increased content of ginsenoside ingredients.

The present invention includes the ginseng fruits treated by the above method.

The present invention further includes the ginseng flower stalks treated by the above method.

The present invention further includes health supplement foods which contain the ginseng fruits with a high content of ginsenoside treated by the above method.

The present invention further includes health supplement foods which contain the ginseng flower stalks with a high content of ginsenoside treated by the above method.

The above health supplement foods may be provided in forms of, for example, liquid drinks, solid tablets, pills, powders, capsules and the like.

The ginseng fruits and/or flower stalks with high content of ginsenoside are preferably contained in the health supplement food according to the present invention an amount of 0.1 to 10 wt.% of the total weight of the health supplement food. The health supplement food may further include additional substances such as, for example, fillers, lubricants, binders, disintegrants, etc. Such additional substances are added to the health supplement foods in forms of, for example, solid tablets, pills, powders, capsules and the like. Since these substances are commonly used and can be appropriately selected by those skilled in the related arts, the present invention does not include detailed description thereof.

The present inventive ginseng fruits and/orftower stalks are contained in the health supplement foods preferably have at least one form selected from the forms including powders, juices, hot water extracts and organic solvent extracts.

The ginseng fruits and/or flower stalks in the powder form are obtained by using a grinder or miller to pulverize the fruits and/or flower stalks,

The ginseng fruits and/or flower stalks in the juice form are obtained by using a screw juicer or a juice extractor to press out the extract of the fruits and/or flower stalks.

The ginseng fruits and/or flower stalks in the hot water extract form are obtained by introducing crude fruits and/or flower stalks in pure water preferably in an amount of from 3 to 10 times the weight of the fruits and/or flower stalks. The mixture is then treated at 80 to 120 °C until the amount of pure water is reduced to 10 to 50 % of the original amount to form an extract. The mixture is then filtered to obtain a filtrate containing the extract and/or concentrating the filtrate to obtain the concentrated product.

The ginseng fruits and/or flower stalks in the organic solvent extract form are obtained by introducing crude fruits and/or flower stalks in the organic solvent preferably in an amount of from 2 to 5 times weight of the fruits and/or flower stalks.

The mixture is then treated at 65 to 75 °C, for 1 to 24 hours, to form an extract. The mixture is then filtered to obtain a filtrate containing the extract and/or concentrating the filtrate to obtain the concentrated product, The organic solvent may be removed from the filtrate through a further removal step.

The organic solvent used to produce the organic solvent extract of the ginseng fruits and/or flower stalks according to the present invention preferably includes alcohol solvents in which the number of carbon atoms ranges from 1 to 20. The organic solvent preferably includes, methanol, ethanol, propanol, butanol and pentanol, more preferably ethanol.

The present invention will now be described in more detail in the following examples.

### EXAMPLE 1-1

The following process is used to produce the ginseng fruits with high contents of ginsenoside ingredients.

Ginseng fruits gathered from ginseng plants underwent infrared irradiation using infrared rays at 100 µm wavelength for 10 hours. 1 kg of the treated fruits with infrared rays were steamed using water vapor at 95 °C for 1 hour and dried at 70 °C.

This steaming and drying process was repeated four times. Then 1 kg of the processed fruits were added to a solution containing a ginsenoside decomposer and treated for 30 minutes in the solution.

The solution containing the ginsenoside decomposer was a solution prepared by adding α-glucosidase as ginsenoside decomposer in an amount of 10 wt.% of the total weight of pure water.

### EXAMPLE 1-2

The ginseng flower stalks with high contents of ginsenoside ingredients were produced in the same manner as in Example 1-1, except that flower stalks gathered from the ginseng plants were used.

### EXAMPLE 2-1

The ginseng fruits with high contents of ginsenoside ingredients were produced in the same manner as in Example 1-1, except that the fruits were subjected to UV irradiation using UV rays at 100 nm wavelength for 5 hours.

### EXAMPLE 2-2

The ginseng flower stalks with high contents of ginsenoside ingredients were produced in the same manner as in Example 2-1, except that flower stalks gathered from the ginseng plants were used.

### EXAMPLE 3-1

The ginseng fruits with high contents of ginsenoside ingredients were produced in the same manner as in Example 1-1, except that the fruits were subjected to visible ray irradiation using visible rays at 500 nm wavelength for 7 hours.

### EXAMPLE 3-2

The ginseng flower stalks with high contents of ginsenoside ingredients were produced in the same manner as in Example 3-1, except that flower stalks gathered from the ginseng plants were used,

### EXAMPLE 4-1

Ginseng fruits gathered from ginseng plants underwent infrared irradiation using infrared rays at 100 µm wavelength for 10 hours.

1 kg of the infrared treated fruits were steamed using water vapor at 95 °C for 1 hour and dried at 70 °C,

This steaming and drying process was repeated four times. Then 1 kg of the processed fruits were added in a solution containing a ginsenoside decomposer and treated for 30 minutes.

1 kg of the decomposer treated fruits additionally underwent ultrasonic treatment at 40 kHz for 1 hour.

The solution containing the ginsenoside decomposer was a solution prepared by adding α-glucosidase as the ginsenoside decomposer in an amount of 10 wt.% of the total weight of pure water.

### EXAMPLE 4-2

The ginseng flower stalks with high contents of ginsenoside ingredients were produced in the same manner as in Example 4-1, except that flower stalks gathered from the ginseng plants were used.

### EXAMPLE 5-1

The ginseng fruits with high contents of ginsenoside ingredients were produced in the same manner as in Example 4-1, except that the fruits were subjected to low frequency treatment at 7 kHz for 1 hour instead of the ultrasonic treatment.

### EXAMPLE 5-2

The ginseng flower stalks with high contents of ginsenoside ingredients were produced in the same manner as in Example 5-1, except that flower stalks gathered from the ginseng plants were used.

### EXAMPLE 6-1

The ginseng fruits with high contents of ginsenoside ingredients were produced in the same manner as in Example 4-1, except that this fruits were subjected to electric field treatment at 15 kV for 3 minutes instead of the ultrasonic treatment.

### EXAMPLE 6-2

The ginseng flower stalks with high contents of ginsenoside ingredients were produced in the same manner as in Example 6-1, except that flower stalks gathered from the ginseng plants were used.

### EXAMPLE 7-1

The ginseng fruits with high contents of ginsenoside ingredients were produced in the same manner as in Example 4-1, except that the fruits were subjected to magnetic field treatment at 20 Gauss for 40 minutes instead of the ultrasonic treatment.

### EXAMPLE 7-2

The ginseng flower stalks with high contents of ginsenoside ingredients were produced in the same manner as in Example 7-1, except that flower stalks gathered from the ginseng plants were used.

### EXPERIMENTAL EXAMPLE 1

Each of the ginseng fruits produced according to Examples 1-1, 2-1, 3-1, 4-1, 5-1, 6-1 and 7-1 was introduced in pure water of five times the total weight of the fruits, and then extracted at 100 °C until the amount of the pure water was reduced to 30 % of the original amount thereof to form a ginseng extract. The resulting extracts from the fruits were used as test samples.

Ginseng roots commercially available in (farmers) markets were extracted in the same manner and condition as described above. The resulting extract was used as a test control.

The test samples and the test control were subjected to measurement of ginsenoside content. The results are shown in the following Table 1.

**TABLE 1:**

| Content of ginsenoside in ginseng extract according to each of Examples 1-1.2-1, 3-1, 4-1, 5-1, 6-1 and 7-1 and the control | |
|---|---|
| Examples | Content of ginsenoside |
| Example 1-1 | 105.4 |
| Example 2-1 | 105.2 |
| Example 3-1 | 105.5 |
| Example 4-1 | 107.6 |
| Example 5-1 | 107.5 |
| Example 6-1 | 107.4 |
| Example 7-1 | 107.6 |
| Control | 100 |

Measured values of the ginsenoside content were determined with reference to ginsenoside Rd as a standard by phase separating the sample into water and butanol, drying the butanol phase, dissolving the dried sample and the standard ginsenoside Rd in glacial acetic acid, adding a 15 wt. % antimony trichloride solution to the prepared solution, heating the mixture to 60°C for 30 minutes, cooling the heated solution and determining absorbance at 525 nm, The content of ginsenoside in the control was defined as 100 and the content of ginsenoside in each of the test samples according to the above examples was determined as a relative ratio to the content of the control. The higher the measured value, the higher the content of ginsenoside.

### EXPERIMENTAL EXAMPLE 2

Each of the ginseng flower stalks produced according to Examples 1-2, 2-2, 3-2, 4-2, 5-2, 6-2 and 7-2 was introduced in pure water of five times the total weight of the flower stalks, and extracted at 100°C until the amount of pure water was reduced to 25 % of the original amount thereof to form a ginseng extract. The resulting extracts from the flower stalks were used as test samples.

Ginseng flower stalks gathered in ginseng farm fields were extracted in the same manner and condition as described above. The resulting extract was used as a test control.

The test samples and the test control were subjected to measurement of ginsenoside content as described in Experimental Example 1, and the results are shown in the following Table 2.

**TABLE 2:**

| Content of ginsenoside in ginseng extract according to each of Examples 1-2, 2-2, 3-3, 4-2, 5-2, 6-2, 7-2 and the control | |
|---|---|
| Examples | Content or ginsenoside |
| Example 1-2 | 104.2 |
| Example 2-2 | 104.1 |
| Example 3-2 | 104.3 |
| Example 4-2 | 106.4 |
| Example 5-2 | 106.3 |
| Example 6-2 | 106.2 |
| Example 7-2 | 106.4 |
| Control | 100 |

### EXPERIMENTAL EXAMPLE 3

The present inventors asked Life Sciences and Chemical Analysis Center of Joongbu University in Korea to analyze the ginsenoside content in the ginseng fruits produced according to Example 1-1 of the present invention, and to compare them with the ginsenoside content in each of black ginseng roots, black ginseng fine roots, red ginseng roots and red ginseng fine roots, all of which were obtained according to Korean Patent No.1 10-0543862. The analysis results are shown in the following Table 3,

**TABLE 3:**

| Analysis results for contents of ginsenoside ingredients in ginseng fruits, compared with conventional ginseng products | | | | | |
|---|---|---|---|---|---|
| Species | Example 1-1 | Black ginseng roots | Black ginseng fine roots | Red ginseng roots | Red ginseng fine roots |
| Ginsenoside Rg3 | 4.51 | 9.18 | 14.23 | 0.18 | 0.10 |
| Ginsenoside Rb1 | 12.98 | 1.78 | 4.78 | 0.69 | 1.37 |
| Ginsenoside Rb2 | 23.11 | 1.51 | 3.99 | 0.19 | 0.71 |
| Ginsenoside Rc | 14.74 | 1.32 | 3.93 | 0.25 | 0.12 |
| Ginsenoside Rd | 31.05 | 1.14 | 5.68 | 0.26 | 0.10 |
| Ginsenoside Re | 135.16 | 0.20 | 1.40 | 0.40 | 0.99 |
| Ginsenoside Rf | - | 0.68 | 1.00 | 0.22 | 0.31 |
| Ginsenoside Rg2 | 3.01 | 0.28 | 0.54 | 0.18 | 0.22 |
| Ginsenoside Rh1 | 2.-22 | 1.06 | 1.17 | confirmed | confirmed |
| Ginsenoside Rh2 | 0.04 | - | - | - | - |
| Ginsenoside X | 0.52 | - | - | - | - |

| | | | | | |
|---|---|---|---|---|---|
| - : not detected Confirmed : detected but not quantified determined values in mg/g | | | | | |

As shown in the result of the Table 3, it was determined that the contents of ginsenoside ingredients in the ginseng fruits produced according to Example 1-1 of the present invention and, especially, the contents of ginsenoside Rb1, ginsenoside Rb2, ginsenoside Rc, ginsenoside Rd, and ginsenoside Re are considerably higher than the corresponding contents in each of black ginseng roots, black ginseng fine roots, red ginseng roots and red ginseng fine roots which were obtained according to Korean Patent No. 10-0543862.

The present invention can produce ginseng fruits and flower stalks with a high content of ginsenoside from ginseng fruits and flower stalks containing abundant saponin.

## Claims

1. A method for producing ginseng fruits and flower stalks with a high content of ginsenoside, comprising the steps of: a) treating the ginseng fruits and flower stalks by light irradiation; b) subjecting the light treated fruits and flower stalks to water vapor then c) drying the vapor treated product: and d) then treating the dried product in a solution containing at least one ginsenoside decomposer.

2. The method according to claim 1, wherein the light is selected from infrared rays, visible rays and ultraviolet (UV) rays,

3. The method according to claim 1 or 2, wherein the ginsenoside decomposer is selected from one or more of Hasegawa bacteria, α-glucosidase, α-Rhamnosidase and soyasapogenol B.

4. The method according to claim 1, 2 or 3, further comprising a treatment process by at least one process selected from ultrasonic treatment, low frequency treatment, electric field treatment and magnetic field treatment after the treatment step d).

5. Fruits obtained according to the method of claim 1.

6. Stalks obtained according to the method of claim 1,

7. Ginseng products comprising an elevated content of at least one of the ginsenoside Rb1, Rb2, Rc, Rd, Re, Rg2 and Rh1. when compared with either one or more of black ginseng roots, black ginseng fine roots, red ginseng roots, and red ginseng fine roots.

8. Health supplement food comprising fruits according to claim 5.

9. Health supplement food comprising stalks according to claim 6.

10. Health supplement food comprising a ginseng product according to claim 7.
